# EUROPEAN PATENT SPECIFICATION

(11) **EP 2 903 514 B1**
(45) Date of publication and mention of the grant of the patent: **23.11.2022**
(21) Application number: 13844071.4
(22) Date of filing: 02.10.2013
(51) Int. Cl.: A61B 5/08, A61M 16/04, A61B 5/00, A61B 5/085, A61B 5/091

(54) **SYSTEMS FOR PULMONARY DIAGNOSIS**
LUNGENDIAGNOSESYSTEME
SYSTÈMES POUR DIAGNOSTIC PULMONAIRE

(30) Priority: 02.10.2012 US 201261709044 P
(43) Date of publication of application: 12.08.2015
(73) Proprietor: Freitag, Lutz, 58675 Hemer (DE); Radhakrishnan, Srikanth, Cupertino, CA 95014 (US)
(72) Inventor: Freitag, Lutz, 58675 Hemer (DE); Radhakrishnan, Srikanth, Cupertino, CA 95014 (US)
(74) Representative: Clark, Jane Anne
(86) International application number: PCT/US2013/063108
(87) International publication number: WO 2014/055675

(56) References cited:
- EP-A1- 0 692 273
- WO-A1-2006/055695
- US-A- 5 313 955
- US-A1- 2011 301 483
- US-A1- 2011 301 483
- US-A1- 2011 313 286
- US-A1- 2012 215 212
- US-B1- 6 398 775
- US-B2- 6 616 625

## Description

### BACKGROUND OF THE INVENTION

1.Field of the Invention. The present disclosure relates generally to medical systems and more specifically to systems for assessing the functionality of lung compartments and treating diseased compartments of the lung.

2. Description of the Related Art. Lung diseases are a problem affecting several millions of people. Chronic obstructive pulmonary disease (COPD), for example, is a significant medical problem affecting 16 million people or about 6% of the U.S. population. In general, two types of diagnostic tests are performed on a patient to determine the extent and severity of lung disease: 1) imaging tests and 2) functional tests. Imaging tests, such as chest x-rays, computed tomography (CT) scans, magnetic resonance imaging (MRI), perfusion scans, and bronchograms, provide a good indicator of the location, homogeneity and progression of the diseased tissue. However, these tests do not give a direct indication of how the disease is affecting the patient's overall lung function and respiration capabilities. This can be measured with functional testing, such as spirometry, plethysmography, oxygen saturation, and oxygen consumption stress testing, among others. Together, these diagnostic tests are used to determine the course of treatment for the patient.

However, the diagnostic tests for COPD are limited in the amount and type of information that may be generated. For example, diagnostic imaging may provide information to the physician regarding which lung regions "appear" more diseased, but in fact a region that appears more diseased may actually function better than one that appears less diseased. Similarly, functional testing is performed on the lungs as a whole. Thus, the information provided to the physician is generalized to the whole lung and does not provide information about functionality of individual lung compartments, which may be diseased. Thus, physicians may find it difficult to target interventional treatments to the compartments most in need and to avoid unnecessarily treating compartments that are least in need of treatment. Therefore, in general, using conventional imaging or functional testing, the diseased compartments cannot be differentiated, prioritized for treatment, or assessed after treatment for their level of response to therapy.

One particular need is the diagnosis of lung compartments that would be candidates for lung volume reduction (LVR). LVR typically involves resecting diseased portions of the lung. Resection of diseased portions of the lungs both promotes expansion of the non-diseased regions of the lung and decreases the portion of air which is inhaled into the lungs but is not used to transfer oxygen to the blood. Lung reduction is conventionally performed in open chest or thoracoscopic procedures where the lung is resected, typically using stapling devices having integral cutting blades. While effective in many cases, conventional lung reduction surgery is significantly traumatic to the patient, even when thoracoscopic procedures are employed. Further, such procedures often result in the unintentional removal of relatively healthy lung tissue or leaving behind of relatively diseased tissue, and frequently result in air leakage or infection.

One of the emerging methods of lung volume reduction involves the endoscopic introduction of implants into pulmonary passageways. Such a method and implant is described in U.S. Patent Application Ser. No. 11/682,986. The implants will typically restrict air flow in the inhalation direction, causing the adjoining lung compartment to collapse over time. This method has been suggested as an effective approach for treating lung compartments that are not subject to collateral ventilation.

There is a need for a quick and convenient method of determining whether a diseased lung portion is suitable for placement of an implant for effective LVR. This depends on the presence of collateral channels which often reduce the effectiveness of LVR using an implant. Collateral channels are sometimes naturally present in the lungs because of gaps in the natural membranes separating the lobes and segments. In many cases, however, COPD manifests itself in the formation of a large number of collateral channels caused by rupture of the air sacs because of hyperinflation, or by destruction and weakening of alveolar tissue, leading to many pathways for air to flow between lung segments. The presence of these collateral channels impedes LVR treatment using one-way valves and implants to induce collapse of a lung segment. This is because the collateral channels allow air to flow into the lung compartment from an adjacent compartment. This replenishes the air in the compartment and prevents the lung compartment from collapsing. If collateral channels exist, options other than LVR may be explored. The selection of this method of LVR as a treatment option would thus be based on the presence or absence of collateral channels. There is thus a need to determine the presence of collateral channels, or at least ventilation due to collateral channels (i.e., collateral ventilation).

Further, if collateral channels are present, regardless of whether LVR is chosen as a treatment option, it would be further desirable to discern their ancillary characteristics, such as the extent of a compartment's hyperinflation, the size of the collateral channels, and the perfusion rate through the pathways and the particular lobes or segments of the lung that are connected by these pathways. Discerning such characteristics enables the treatment to be tailored to the nature and quality of the collateral channels. For example, depending on the nature and size of the collateral channels, different agents may have to be used to seal the collateral channels. There is therefore a need for accurately determining the presence of collateral pathways as well as the characteristics of such pathways.

Various methods for determining collateral ventilation have been proposed. For example, Morrel et al. (1994) analyzed gas compositions in lungs of emphysematous patients. After occluding a lung compartment, they introduced an O₂-He mixture as a breathing gas into the isolated lung compartments. The helium gas content in the isolated lung was measured, as was the CO₂ content. They correlated the rise of helium within the isolated compartment to the extent of collateral ventilation. They also measured significantly lower P_{CO2}, in the occluded segments in emphysematous patients, but could not conclude definitively on the state of collateral ventilation using these measurements.

More recently, a number of methods for determining collateral ventilation have been disclosed, as in co-pending U.S. Published Patent Applications 2003/0051733, 2003/0055331, 2007/0142742, 2006/0264772 and 2008/0200797. U.S. Patent Application 2003/0055331 discloses a non-invasive method of diagnosing the presence of disease in various parts of the lung using imaging and computerized integration of the imaging data. The methods described help determine which lung portions are the most severely affected and which lung channels will respond effectively to isolation treatment.

An endobronchial catheter-based diagnostic system is disclosed in U.S. Patent Application 2003/0051733, wherein the catheter uses an occlusion member to isolate a lung segment and the instrumentation is used to gather data such as changes in pressure and volume of inhaled/exhaled air. The data collected is used to diagnose the extent of hyperinflation, lung compliance, etc., in the lung segment. The Application also discloses the use of radiopaque gas and polarized gas that would enable the presence of collateral channels to be identified using radiant imaging and MRI, respectively. A similar method is disclosed in U.S. Patent Application 2008/0027343 in which an isolation catheter is used to isolate a targeted lung compartment and pressure changes therein are sensed to detect the extent of collateral ventilation. Other applications that disclose methods of diagnosis of collateral ventilation include U.S. Patent Application 2007/0142742, U.S. Patent Application 2005/0288702, U.S. Patent Application 2006/0264772, U.S. Patent Application 2008/0200797, U.S. Patent Application 2006/0276807.

US 2011/0301483 describes a method of determining potential treatment sites in a diseased lung in which an assessment catheter is introduced into a lung passageway. The catheter has a distal portion comprising an occluding member and a proximal portion configured to operatively mate with an external console. The catheter is used to identify one or more assessment sites within the airways of the lung. At each assessment site, at least one physiological, anatomical or biological characteristic is determined. A characteristic score for each assessment site is calculated based on a predetermined algorithm and a treatment is determined based on the scores of the assessment sites.

US 6,398,775 describes apparatus, systems, methods and kits for isolating a target lung segment and treating that segment, usually by drug delivery or lavage. The systems include at least one lobar or sub-lobar isolation catheter which is introduced beyond a second lung bifurcation and which can occlude a bronchial passage at that point. An inner catheter is usually introduced through the isolation catheter and is used in cooperation with the isolation catheter for delivering and/or removing drugs or washing liquids from the isolated lung region. Optionally, the inner catheter will also have an occluding member near its distal end for further isolation of a target region within the lung.

WO 2006/055695 describes a device and method for lung treatment and in particular for affecting lung volume reduction by delivering a condensable vapor at a temperature above body temperature to the desired regions of the patient's lung to damaged tissue therein.

EP 0692273 described apparatus for delivering a medicine to a patient via the patient's respiratory system. A nebulization catheter is positioned in the patient's respiratory system so that a distal end of the nebulization catheter is in the respiratory system and a proximal end is outside the body. The nebulization catheter may be used in conjunction with an endotracheal tube and preferably is removable from the endotracheal tube.

### SUMMARY OF THE INVENTION

The present invention is set out in the appended claims.

Aspects of present disclosure comprise a pulmonary diagnostic system with a pulmonary catheter whose distal end comprises an inflatable occlusion balloon. In one aspect, the pulmonary diagnostic system further comprises a hollow insert configured to be insertable and removable from an innermost lumen of the pulmonary catheter, wherein the hollow insert reduces the inner diameter of the pulmonary catheter. In another aspect, the system also comprises a sensor that is in fluid communication with the insert and a pulmonary diagnostic device. The sensor is configured to generate measurement data reflecting a respiratory or physiological feature of a lung compartment. The pulmonary diagnostic device is configured to transfer fluid from the lung, measure data received from at least one sensor, and process and display measurement data received from the sensor.

In another aspect, a pulmonary diagnostic system having a pulmonary catheter with a distal end comprises an inflatable occlusion balloon and a sensor in fluid communication with the pulmonary catheter. In one aspect, a pulmonary diagnostic system comprises an access port whereby a user may apply vacuum to evacuate a fluid within a lumen of the catheter. In another aspect, the system also comprises a pulmonary diagnostic device configured to transfer fluid or gas from the lung, measure data received from at least one sensor, and process and display measurement data received from the sensor. In yet another aspect, the sensor is configured to generate measurement data reflecting a respiratory or physiological feature of a lung compartment.

Also disclosed are methods of diagnosing a lung compartment. In aspect, the method comprising inserting a balloon pulmonary catheter into a lung compartment, wherein a lumen of the catheter comprises a removable hollow insert. Thereafter measurement data reflecting a physiological feature of a lung compartment is detected using a sensor, and a characteristic of a lung compartment is calculated using a pulmonary diagnostic device. The hollow insert is configured to be insertable and removable from an innermost lumen of the pulmonary catheter, and the hollow insert reduces the inner diameter of the pulmonary catheter.

In another aspect, a method of diagnosing a lung compartment comprises inserting a catheter into a lung compartment and applying a vacuum to the catheter to remove the volume of air within an inner lumen of the catheter. In one aspect, measurement data reflecting a physiological feature of a lung compartment is detected using a sensor. In another aspect, a characteristic of a lung compartment is then calculated using a pulmonary diagnostic device.

### BRIEF DESCRIPTION OF THE DRAWINGS

The invention has other advantages and features which will be more readily apparent from the following detailed description of the invention and the appended claims, when taken in conjunction with the accompanying drawings, in which:
**FIG.** 1 shows one embodiment of an existing pulmonary catheter.
**FIG. 2A** shows one embodiment of a pulmonary diagnostic system comprising a pulmonary catheter and a removable insert.
**FIG. 2B** shows one embodiment of a pulmonary diagnostic system comprising a pulmonary catheter with a removable insert placed within the catheter.
**FIG. 3** shows one embodiment of an endobronchial Pulmonary Diagnostic device.
**FIG. 4** shows a flow diagram illustrating one method of using one embodiment of the pulmonary diagnostic system.
**FIG. 5** shows one embodiment of a pulmonary diagnostic system comprising a pulmonary catheter with an access port.

### DETAILED DESCRIPTION

While the invention has been disclosed with reference to certain embodiments, it will be understood by those skilled in the art that various changes may be made and equivalents may be substituted without departing from the scope of the invention as claimed. In addition, many modifications may be made to adapt to a particular situation or material to the teachings of the invention without departing from its scope as claimed.

Throughout the specification and claims, the following terms take the meanings explicitly associated herein unless the context clearly dictates otherwise. The meaning of "a", "an", and "the" include plural references. The meaning of "in" includes "in" and "on." Referring to the drawings, like numbers indicate like parts throughout the views. Additionally, a reference to the singular includes a reference to the plural unless otherwise stated or inconsistent with the disclosure herein.

The word "exemplary" is used herein to mean "serving as an example, instance, or illustration." Any implementation described herein as "exemplary" is not necessarily to be construed as advantageous over other implementations.

Throughout this description, reference is made to the term "lung compartment". As used herein, the term "lung compartment" refers to a defined division or portion of a lung. For purposes of example, lung compartments are described herein with reference to human lungs, wherein some exemplary lung compartments include lung lobes and lung segments. Thus, the term "lung compartment" as used herein can refer, for example, to a lung lobe or a lung segment. Such nomenclatures conform to nomenclature for portions of the lungs that are known to those skilled in the art. However it should be appreciated that the term lung compartment does not necessarily refer to a lung lobe or a lung segment, but can refer to some other defined division or portion of a human or non-human lung.

In general certain terms are used that refer to relative directions or locations along a path defined from an entryway into the patient's body (e.g., the mouth or nose) to the patient's lungs. The path of airflow into the lungs generally begins at the patient's mouth or nose, travels through the trachea into one or more bronchial passageways, and terminates at some point in the patient's lungs. The term "proximal direction" refers to the direction that point toward the patient's mouth or nose and away from the patient's lungs. In other words, the proximal direction is generally the same as the expiration direction when the patient breathes. The term "distal direction" refers to the direction that point towards the patient's lung and away from the mouth or nose. The distal direction is generally the same as the inhalation or inspiratory direction when the patient breathes.

As used herein the term "direct pathway" refers to a bronchial passageway that branches directly or indirectly from the trachea and either terminates in the targeted lung compartment to thereby directly provide air to the targeted lung compartment; or branches into at least one other bronchial passageway that terminates in the targeted lung compartment to thereby directly provide air to the targeted lung compartment. The term "collateral pathway" refers to any pathway that provides air to the targeted lung compartment and that is not a direct pathway. The term "direct" is used to refer to air flow that flows into or out of a targeted lung compartment via a direct pathway. Likewise, the term "collateral" is used to refer to fluid flow (such as air flow) that flows into or out of a targeted lung compartment via a collateral pathway. Thus, for example, "direct" flow is fluid flow (such as air flow) that enters or exits the targeted lung compartment via a direct pathway, and "collateral" flow is fluid flow (such as air flow) that enters or exits the targeted lung compartment via a collateral pathway.

Present disclosure relates to non-invasive devices, systems, and methods of diagnosing a state of a lung compartment including but not limited to the presence of disease in various parts of the lung or which lung channels will respond effectively to isolation as well as other treatments. Specifically, the present disclosure contemplates devices, systems, and methods for diagnosing a lung compartment using a multi-lumen pulmonary diagnostic catheter configured to minimize a collection of a volume of fluid that resides within the catheter prior to the sample collection from the lung compartment.

Typically, a bronchial diagnostic system comprises a pulmonary diagnostic catheter that is used to collect a sample from a lung compartment. In one embodiment, a pulmonary diagnostic catheter comprises a proximal end and a distal end, wherein the distal end comprises occlusion elements that is configured to at least partially sealingly engage an airway feeding a lung compartment.

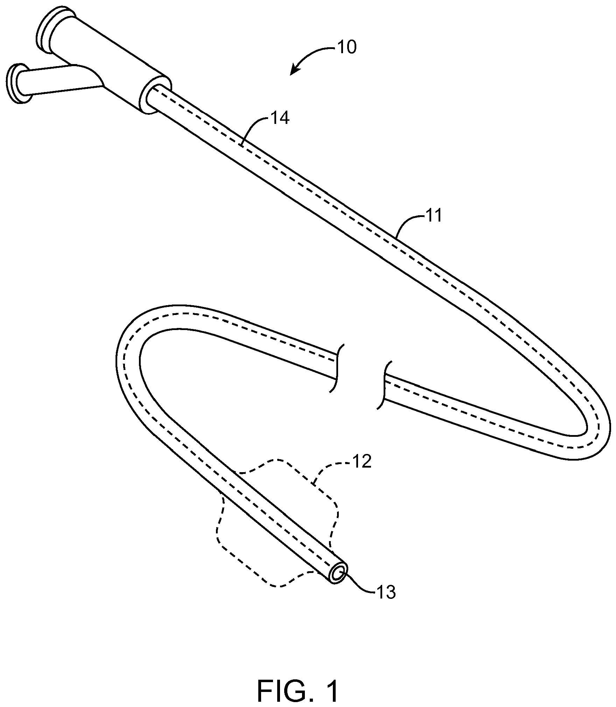

Embodiments of pulmonary diagnostic catheters have been disclosed in the co-pending U.S. Patent Publication No 2010/0158795 assigned to the same assignee. In particular, one embodiment of a pulmonary diagnostic catheter is shown in FIG. 1, where the pulmonary catheter **10** comprises a catheter body **11,** an expandable occluding member **12** on the catheter body, and a mechanism for inflating the occluding member (not shown). The catheter body **11** usually has a distal end, a proximal end, and at least one lumen **13** extending from a location at or near the distal end to a location at or near the proximal end. The expandable occluding member **12** is disposed near the distal end of the catheter body and is adapted to be expanded in the airway which feeds the target lung compartment. In the embodiment shown in FIG. 1, air exchange between the proximal and distal ends of the catheter occurs via the lumen **13.**

A problem is presented in that there may be a volume of pre-existing gas that resides within the lumen **13** prior to the beginning of the measurements. Specifically, the pulmonary catheter **10** inherently contains some volume of air that remains within the lumen **13** when the catheter is inserted into the lung. When diagnostic measurements begin, part of the initial measurements will be compromised by the presence of this pre-existing gas, also referred to herein as the dead space air. This is because the air flowing through the measuring components of the device will initially be the dead space air. The user of the measuring device would thus have little to no idea of when the measurements taken would reflect the characteristics of the air coming from the lungs of the patient rather than the dead space air within the catheter.

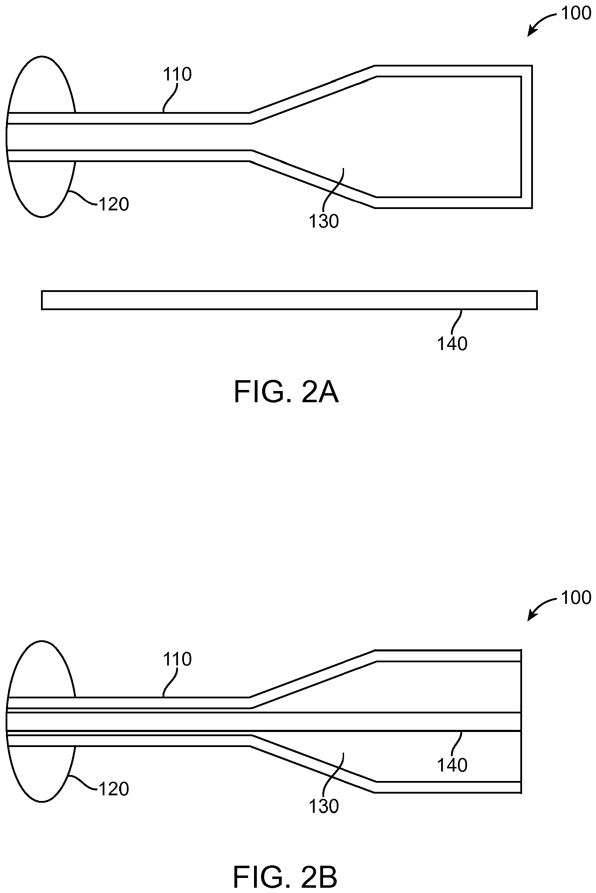

In one embodiment, as seen in FIGS. 2A and 2B, a pulmonary catheter system **100** comprises a pulmonary diagnostic catheter **110** with an expandable occluding member **120** disposed on the catheter body and a mechanism for inflating the occluding member (not shown), and a catheter lumen **130** disposed between the distal and proximal ends of the catheter **110.**

In one embodiment, at least a distal portion of the pulmonary diagnostic catheter **110** is adapted to be advanced into and through the trachea. The distal end of the catheter **110** can then be directed to a lung lobe to reach an airway which feeds a target lung compartment that is to be assessed and/or treated. When the occluding member **120** is expanded, the target lung compartment is isolated with access provided only through the lumen **130** or catheter body when the occluding member **120** is expanded. The targeted lung compartment is fed through a "direct pathway" or" collateral pathway".

The pulmonary catheter system **100** further comprises at least one removable insert **140.** In one embodiment, the insert **140** is configured as substantially hollowed and it is configured to reside within the catheter lumen **130** such that the insert **140** functions as an innermost lumen of the pulmonary catheter **110.** It is contemplated that the insert **140** may be insertable and/or removable from the lumen **130.** Alternatively, the insert **140** may be incorporated as a part of the pulmonary catheter **110.** In one embodiment, the insert **140** extends substantially the length of the pulmonary catheter **110** between the distal and the proximal ends to allow fluid communication such as air flow from the distal end to the proximal end of the catheter **110** through the insert **140.** In another embodiment, the insert **140** may be longer than the length of the pulmonary catheter **110,** wherein a portion of the insert **140** may extend beyond the distal end of the catheter **110.** In one embodiment, the insert **140** may be a unibody insert. In another embodiment, the insert **140** may be a telescoping insert, wherein the insert comprises multiple segments such that one segment may be extended from another segment to achieve the desired length of the insert.

In one embodiment, the insert **140** is configured with a smaller volume than the lumen **130,** wherein any dead space air that resides within the insert **140** is less than the dead space air that resides within the lumen **130.** In such an embodiment, the user of the measuring device is capable of obtaining a sample from the lung through the insert **140** of the catheter **110,** wherein the likelihood and the amount of the initial air sample taken via the insert **140** containing dead space air is decreased due to the smaller volume of the insert **140** while maintaining the volume and dimension of the catheter lumen **130.** This configuration is advantageous in that the volume and dimension of the catheter lumen **130** may be configured to be of sufficient size to allow insertions of additional equipments through the lumen **130.**

The pulmonary diagnostic catheter **110** may take a variety of forms, each suitable for acquiring measurement data to characterize the lung compartment or to perform a treatment on the lung compartment. In addition, the pulmonary catheter may have a number of additional features, such as guidewire lumen, optical imaging capability and steering capability, to name a few. In one embodiment, the dimensions of the pulmonary diagnostic catheter **110** may be configured to facilitate the insertion and/or removal of the diagnostic catheter **110** to the lung while the insert **140** maintains the volume configured to minimize dead space air being collected during the initial sample collection. In embodiment, the dimensions and materials of the pulmonary catheter **110** are selected to permit endotracheal introduction and intraluminal advancement through the lung bronchus, optionally over a guide wire, and/or through a primary tracheal tube structure and/or inside the working channel of a bronchoscope. Alternatively, the catheter **110** may assumes various other dimensions dependent the intended use on the patient anatomy.

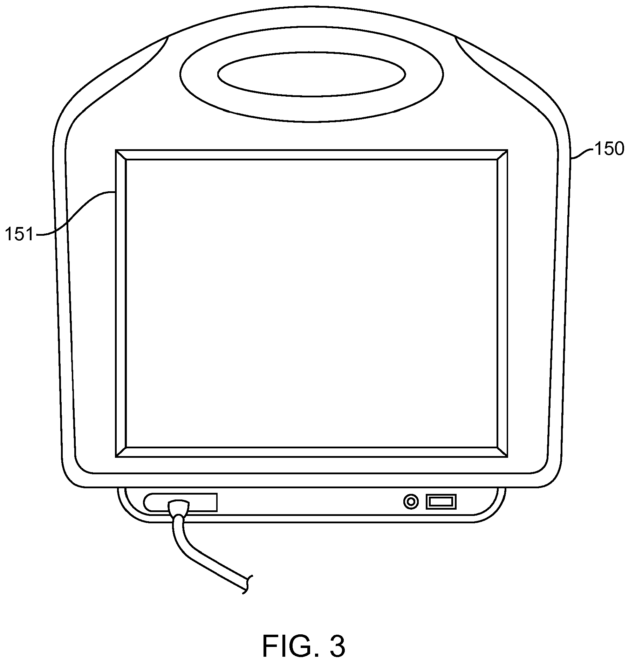

In one embodiment, the proximal end of the catheter **110** is associated with a endobronchial pulmonary diagnostic device **150,** as shown in FIG. 3 and as disclosed in the co-pending U.S. Patent Publication No 2010/0158795 assigned to the same assignee. The pulmonary diagnostic device **150** comprises mechanisms to measure lung functionality. Pulmonary diagnostic device **150** may also comprise mechanisms to introduce or remove a gas or any agent into the isolated lung compartment via the body lumen **130.** The pulmonary diagnostic device **150** will typically comprise an interface for receiving input from a user and a display screen **151.** The display-screen **151** will optionally be a touch-sensitive screen, and may display preset values. Optionally, the user may input information into the pulmonary diagnostic device **150** via a touch-screen mechanism.

Additionally, in one embodiment, the pulmonary catheter system **100** further comprises at least one sensor, for example, a sensor that resides within a sensor compartment that is in fluid communication with the pulmonary catheter and/or the insert **140.** The sensor may be configured to generate measurement data reflecting a physiological feature of a lung compartment. The sensor compartment maybe discrete from the catheter **110** and/or the isnert **140,** disposed on or within the pulmonary catheter **110** and/or the insert **140.** Alternatively, such a sensor may be disposed anywhere in the system, including with the Pulmonary diagnostic device **150** or within another connected devices or components. In one embodiment, the sensor is disposed within a sensor compartment, wherein the sensor compartment is in fluid communication with the insert **140.**

Generally, the sensor is configured to gather measurement data or information to generate respiratory or physiological feature of a lung compartment which is transmitted to the pulmonary diagnostic device **150.** The diagnostic device **150** has a mechanism for receiving the measurement data. Often, the diagnostic device also comprises mechanisms for processing the measurement data. Processing may comprise converting the measurement data into a form which may be visually displayed, such as in graphs, charts, tables, numbers, images or figures. Additionally and optionally, the processing may comprise analyzing the data wherein the data is used to determine or calculate secondary information or data such as an average pressure value, a volume value, a compliance value, an average tidal volume value and/or a resistance value, to name a few. Alternatively, processing may comprise converting the measurement data into a computer readable format. Such conversion may be of the measurement data itself or of secondary data derived from the measurement data.

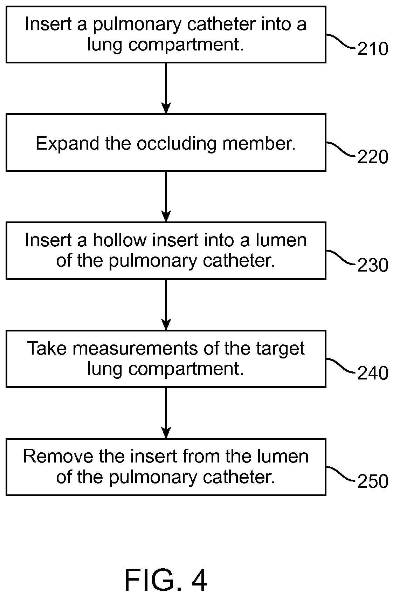

Referring now to FIG. 4 where a flow diagram illustrating the steps of one method of diagnosing a lung compartment. At step **210,** the method involves inserting a pulmonary catheter as shown in FIG. 2 into a lung compartment. In one embodiment, the catheter **110** is advanced into and through the trachea. The distal end of the catheter is then directed to a lung lobe to reach an airway which feeds a target lung compartment that is to be assessed and/or treated. At step **220,** after the catheter reached the airway which feeds a target lung compartment, the occluding member **130** is expanded whereby the target lung compartment is isolated such that access is provided only through the lumen **130** or catheter body.

At step **230,** a hollow insert **140** is inserted into the lumen **130** such that it allows fluid communication between the airway and at least one sensor through the hollow insert **140.** Alternatively, in an embodiment where the insert is integrated into the catheter, the insertion step is not performed.

At step **240,** various measurements of the target lung compartment may be measured. Particularly, measurement data reflecting a respiratory or physiological feature of a lung compartment is detected using the sensor and a characteristic of a lung compartment may then be calculated using a pulmonary diagnostic device. At step **250,** the insert **140** is removed from the lumen **130.** In one embodiment, the insert **140** may be removed after the initial measurement. In another embodiment, the insert **140** may be removed after the completion of the all measurement and diagnosis activities. In yet another embodiment, the insert **140** may be removed at during various stages of the procedure.

It is further contemplated that various other devices and/or equipments may be inserted to the lumen **130** to access, diagnose, or treat the target lung compartment before or after the insertion of the hollow insert **140.**

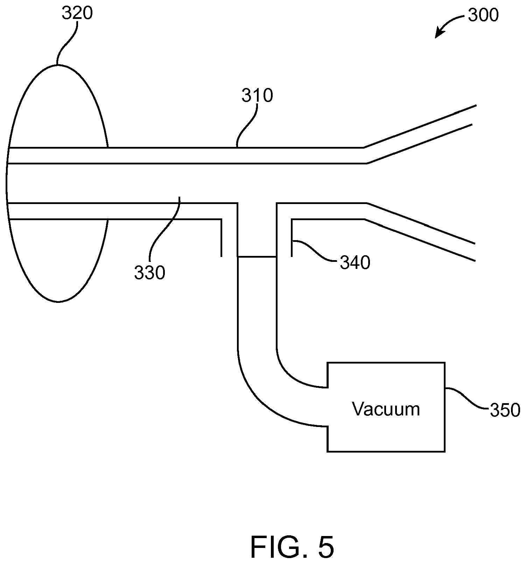

Referring now to FIG. 5, where another embodiment of a pulmonary catheter system is exemplarily shown. As seen in FIG. 5, the pulmonary catheter system **300** comprises a pulmonary catheter **310** with an expandable occluding member **320** disposed on the catheter body and a mechanism for inflating the occluding member (not shown). The pulmonary catheter further comprises a catheter lumen **330** disposed between the distal and proximal ends of the catheter **310.** The pulmonary catheter **310** further comprises at least one access port **340** disposed on the pulmonary catheter **310,** wherein the access port **340** is configured to enable fluid communication with the catheter lumen **330.** The access port **340** may be connected to a vacuum source **350** wherein a fluid, such as dead space air that resides within the catheter lumen **310** may be evacuated, thus substantially reducing or eliminating the dead space air from the catheter lumen **310.**

In one embodiment, the access port **340** may be connected to a directional fluid restrictive device such as a one-way valve to prevent fluid flow into the lumen **330.** The vacuum source **350** may be a fixed volume pump or other vacuum source to apply controlled vacuum to aspirate or evacuate the lumen **330.** Additionally, the access port **340** may function as a sampling port such that a fluid sample may be collected from or at the access port **340.** In one embodiment, the access port **340** is connected to a sensor compartment (not shown), wherein at least one sensors as previously described resides. In another embodiment, the vacuum source **350** may be connected to the sensor compartment wherein the vacuum is applied via the sensor compartment.

Various embodiments of the inserts and the catheters as described above may be constructed of various suitable materials include low and high density polyethylenes, polyamides, nylons, PTFE, PEEK, and the like. The occlusion balloon can be made from elastomeric materials, such as polyurethane, low density polyethylene, polyvinylchloride, silicone rubber, latex, and the like.

Present disclosure further contemplates kits comprise a pulmonary diagnostic catheter, an insert, and instructions for the use thereof. Optionally, the kits may further include any of the other system components described above, such as at least one sensor, a mechanism for inflating the occluding member, a vacuum source or other components. The instructions for use will set forth any of the methods as described above, and all kit components will usually be packaged together in a pouch or other conventional medical device packaging. Usually, those kit components, such as a pulmonary diagnostic catheter, an insert, which will be used in performing the procedure on the patient will be sterilized and maintained sterilely within the kit. Optionally, separate pouches, bags, trays, or other packaging may be provided within a larger package, where the smaller packs may be opened separately and separately maintain the components in a sterile fashion.

While the above is a complete description of the preferred embodiments of the invention, various alternatives, modifications, and equivalents may be used. Therefore, the above description should not be taken as limiting the scope of the invention which is defined by the appended claims.

## Claims

1. A pulmonary diagnostic system comprising:
a pulmonary catheter (110) having a proximal end and a distal end, wherein the distal end comprises an inflatable occlusion balloon configured to isolate a lung compartment;
a hollow insert (140) configured to be insertable and removable from a lumen of the pulmonary catheter, wherein the hollow insert reduces the inner diameter of the pulmonary catheter such that dead space air that resides within the hollow insert (140) is less than dead space air that resides within the lumen of the pulmonary catheter;
a sensor in fluid communication with an airway through the hollow insert, said sensor configured to generate measurement data reflecting a physiological feature of the isolated lung compartment; and
a pulmonary diagnostic device configured to transfer fluid from the isolated lung compartment, measure data received from at least one sensor, and process and display measurement data received from the sensor.

2. The pulmonary diagnostic system of claim 1, wherein the sensor resides in a sensor compartment that is in communication with the pulmonary diagnostic device.

3. The pulmonary diagnostic system of claim 2, wherein the sensor compartment is configured to allow a user to create a vacuum within a lumen of the catheter.

4. A pulmonary diagnostic system comprising:
a pulmonary catheter (310) having a proximal end and a distal end, wherein the distal end comprises an inflatable occlusion balloon (320) configured to isolate a lung compartment;
an access port (340) that is in fluid communication with a lumen (330) of the pulmonary catheter (310) and a vacuum source (350);
a sensor in communication with the pulmonary catheter (310), the sensor residing in a sensor compartment that is in fluid communication with the lumen (330); and
a pulmonary diagnostic device configured to transfer fluid from the isolated lung compartment, measure data received from at least one sensor, and process and display measurement data received from the sensor, wherein the access port (340) is configured to be connectable to a sensor compartment and wherein the sensor compartment is configured to be connectable to the vacuum source (350); wherein the vacuum source is configured to remove dead space air that resides within the pulmonary catheter.

5. The pulmonary diagnostic system of claim 4, wherein the sensor is configured to generate measurement data reflecting a physiological feature of the isolated lung compartment.

6. The pulmonary diagnostic system of claim 4, further comprising a hollow insert (140) configured to be insertable and removable from an innermost lumen of the pulmonary catheter, wherein the hollow insert reduces the inner diameter of the pulmonary catheter such that dead space air that resides within the hollow insert (140) is less than dead space air that resides within the lumen of the pulmonary catheter.

## Patentansprüche

1. Pulmonaldiagnosesystem, das Folgendes umfasst:
einen Pulmonalkatheter (110) mit einem proximalen Ende und einem distalen Ende, wobei das distale Ende einen aufblasbaren Okklusionsballon umfasst, der dafür konfiguriert ist, ein Lungenkompartiment zu isolieren;
einen hohlen Einsatz (140), der dafür konfiguriert ist, in ein Lumen des Pulmonalkatheters eingeführt und aus diesem entfernt zu werden, wobei der hohle Einsatz den Innendurchmesser des Pulmonalkatheters so reduziert, dass die Totraumluft, die sich in dem hohlen Einsatz (140) befindet, geringer ist als die Totraumluft, die sich in dem Lumen des Pulmonalkatheters befindet;
einen Sensor in Fluidverbindung mit einem Luftweg durch den hohlen Einsatz, wobei der genannte Sensor dafür konfiguriert ist, Messdaten zu erzeugen, die ein physiologisches Merkmal des isolierten Lungenkompartiments widerspiegeln; und
eine Pulmonaldiagnosevorrichtung, die dafür konfiguriert ist, Flüssigkeit aus dem isolierten Lungenkompartiment zu transferieren, von mindestens einem Sensor empfangene Daten zu messen und von dem Sensor empfangene Messdaten zu verarbeiten und anzuzeigen.

2. Pulmonaldiagnosesystem nach Anspruch 1, wobei sich der Sensor in einem Sensorfach befindet, das mit der Pulmonaldiagnosevorrichtung in Verbindung steht.

3. Pulmonaldiagnosesystem nach Anspruch 2, wobei das Sensorfach dafür konfiguriert ist, einem Benutzer zu ermöglichen, ein Vakuum in einem Lumen des Katheters zu erzeugen.

4. Pulmonaldiagnosesystem, das Folgendes umfasst:
einen Pulmonalkatheter (310) mit einem proximalen Ende und einem distalen Ende, wobei das distale Ende einen aufblasbaren Okklusionsballon (320) umfasst, der dafür konfiguriert ist, ein Lungenkompartiment zu isolieren;
eine Zugangsöffnung (340), die in Fluidverbindung mit einem Lumen (330) des Lungenkatheters (310) und einer Vakuumquelle (350) steht;
einen Sensor, der mit dem Pulmonalkatheter (310) in Verbindung steht, wobei sich der Sensor in einem Sensorfach befindet, das in Fluidverbindung mit dem Lumen (330) steht; und
eine Pulmonaldiagnosevorrichtung, die dafür konfiguriert ist, Flüssigkeit aus dem isolierten Lungenkompartiment zu transferieren, von mindestens einem Sensor empfangene Daten zu messen und von dem Sensor empfangene Messdaten zu verarbeiten und anzuzeigen, wobei die Zugangsöffnung (340) dafür konfiguriert ist, mit einem Sensorfach verbunden zu werden, und wobei das Sensorfach dafür konfiguriert ist, mit der Vakuumquelle (350) verbunden werden zu können; wobei die Vakuumquelle dafür konfiguriert ist, Totraumluft zu entfernen, die sich innerhalb des Pulmonalkatheters befindet.

5. Pulmonaldiagnosesystem nach Anspruch 4, wobei der Sensor dafür konfiguriert ist, Messdaten zu erzeugen, die ein physiologisches Merkmal des isolierten Lungenkompartiments widerspiegeln.

6. Pulmonaldiagnosesystem nach Anspruch 4, das ferner einen hohlen Einsatz (140) umfasst, der dafür konfiguriert ist, in ein innerstes Lumen des Pulmonalkatheters eingeführt und aus diesem entfernt zu werden, wobei der hohle Einsatz den Innendurchmesser des Pulmonalkatheters so reduziert, dass die Totraumluft, die sich in dem hohlen Einsatz (140) befindet, geringer ist als die Totraumluft, die sich in dem Lumen des Pulmonalkatheters befindet.

## Revendications

1. Système de diagnostic pulmonaire comprenant:
un cathéter pulmonaire (110) ayant une extrémité proximale et une extrémité distale, l'extrémité distale comprenant un ballon d'occlusion gonflable configuré pour isoler un compartiment pulmonaire;
un insert creux (140) configuré pour pouvoir être inséré dans et retiré d'une lumière du cathéter pulmonaire, l'insert creux réduisant le diamètre intérieur du cathéter pulmonaire de telle sorte que l'air de l'espace mort présent dans l'insert creux (140) est inférieur à l'air de l'espace mort présent dans la lumière du cathéter pulmonaire;
un capteur en communication fluide avec une voie respiratoire par l'intermédiaire de l'insert creux, ledit capteur étant configuré pour générer des données de mesure reflétant une caractéristique physiologique du compartiment pulmonaire isolé; et
un dispositif de diagnostic pulmonaire configuré pour transférer un fluide à partir du compartiment pulmonaire isolé, mesurer des données reçues en provenance d'au moins un capteur, et traiter et afficher les données de mesure reçues en provenance du capteur.

2. Système de diagnostic pulmonaire selon la revendication 1, dans lequel le capteur est présent dans un compartiment de capteur qui est en communication avec le dispositif de diagnostic pulmonaire.

3. Système de diagnostic pulmonaire selon la revendication 2, dans lequel le compartiment de capteur est configuré pour permettre à un utilisateur de créer un vide à l'intérieur d'une lumière du cathéter.

4. Système de diagnostic pulmonaire comprenant:
un cathéter pulmonaire (310) ayant une extrémité proximale et une extrémité distale, l'extrémité distale comprenant un ballon d'occlusion gonflable (320) configuré pour isoler un compartiment pulmonaire;
un orifice d'accès (340) qui est en communication fluide avec une lumière (330) du cathéter pulmonaire (310) et une source de vide (350);
un capteur en communication avec le cathéter pulmonaire (310), le capteur étant présent dans un compartiment de capteur qui est en communication fluide avec la lumière (330); et
un dispositif de diagnostic pulmonaire configuré pour transférer un fluide à partir du compartiment pulmonaire isolé, mesurer des données reçues en provenance d'au moins un capteur, et traiter et afficher les données de mesure reçues en provenance du capteur, l'orifice d'accès (340) étant configuré pour pouvoir être connecté à un compartiment de capteur et le compartiment de capteur étant configuré pour pouvoir être connecté à la source de vide (350); dans lequel la source de vide est configurée pour retirer l'air de l'espace mort qui est présent dans le cathéter pulmonaire.

5. Système de diagnostic pulmonaire selon la revendication 4, dans lequel le capteur est configuré pour générer des données de mesure reflétant une caractéristique physiologique du compartiment pulmonaire isolé.

6. Système de diagnostic pulmonaire selon la revendication 4, comprenant en outre un insert creux (140) configuré pour pouvoir être inséré dans et retiré d'une lumière située tout à l'intérieur du cathéter pulmonaire, l'insert creux réduisant le diamètre intérieur du cathéter pulmonaire de telle sorte que l'air de l'espace mort présent dans l'insert creux (140) est inférieur à l'air de l'espace mort qui est présent dans la lumière du cathéter pulmonaire.
